# EUROPEAN PATENT APPLICATION

(11) **EP 4 596 101 A1**
(43) Date of publication of application: **06.08.2025**
(21) Application number: 23870778.0
(22) Date of filing: 26.09.2023
(51) Int. Cl.: B01J 23/16, B01J 32/00, B01J 35/00, B01J 37/08, C07C 4/08

(54) **SOLID ACID CATALYST, PREPARATION THEREFOR, AND USE THEREOF**

(30) Priority: 26.09.2022 CN 202211177148
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: WU, Yanan, Shanghai 201208 (CN); LI, Xuguang, Shanghai 201208 (CN); LI, Jingqiu, Shanghai 201208 (CN); QI, Xiaolan, Shanghai 201208 (CN); KONG, Dejin, Shanghai 201208 (CN)
(74) Representative: karo IP
(86) International application number: PCT/CN2023/121407
(87) International publication number: WO 2024/067549

(57) **Abstract**

Disclosed are a solid acid catalyst, and preparation and use thereof, wherein the catalyst comprises: a) a metal oxide carrier; and b) at least one modifying metal component; wherein, when the catalyst is characterized by solid nuclear magnetic resonance with trimethylphosphine as the probe molecule, it exhibits a spectral peak in the range of -5 to -20 ppm. The catalyst can be used for hydrocracking reaction of non-aromatic hydrocarbons, and has the advantages of high conversion rate and low side reactions.

## Description

### Technical Field

The present application relates to the field of acid catalysis, and specifically to a solid acid catalyst, and preparation and use thereof.

### Background Art

Lewis acid (Lewis acid) refers to a chemical species with an empty orbital and capable of accepting electrons. For example, the metal ion sites on the surface of a metal oxide with unsaturated-coordination are potential Lewis acid sites. The Lewis acid sites in silica-alumina molecular sieves are closely related to Al, and Al sites with empty orbitals can participate in reactions as Lewis acid sites in molecular sieves. Lewis acids are widely used in various catalytic fields. During reaction processes, they can not only independently catalyze the reactions, but also can cooperate with other active sites such as Bronsted acid sites, metal species, alkaline sites and the like to obtain the target products. The formation of C-C bonds catalyzed by Lewis acids is an important method for C-C bond construction in classical organic synthesis, and both F-C reaction (Friedel-Crafts reaction) and D-A reaction (Diels-Alder reaction) can be performed by the catalysis of the most common Lewis acid catalysts such as AlCl₃, TiCl₄ and the like. Lewis acid-base catalyst pair, which is composed of a Lewis acid and a Lewis base, can catalyze reactions of small molecules to generate polymers and has received great attention in the fields of polymer synthesis and the like. In addition to the field of thermal catalysis, Lewis acids are also widely used in the fields of electro catalysis and photocatalysis. For example, hindered Lewis acid-base pairs can promote the highly selective electrocatalytic urea production process, and Lewis acid sites and photosensitive centers can function jointly to achieve efficient photoredox reactions. Research on Lewis acid sites has attracted more and more attention. The strength of Lewis acids reflects the ability of the active sites to accept electrons during the reaction, which is closely related to the diffusion of molecules and the reaction process on the catalyst surface. Therefore, the regulating of the strength of the Lewis acid on the catalyst surface is an important way to control its reaction performance.

CN108,262,057A discloses a catalyst system composed of a molecular sieve, a metal adjuvant and a binder. The catalyst system improves the cracking activity of the catalyst by adjusting the acid distribution in the catalyst, which can increase the conversion rate of non-aromatic hydrocarbons and selectivity.

CN105,272,804A discloses a method for increasing xylene production by aromatic hydrocarbon transalkylation and non-aromatic hydrocarbon cracking. The method uses Beta molecular sieve as the main active component, and designs a double-layer catalyst system. The non-aromatic hydrocarbon component is subjected to selective ring-opening cracking to generate C₂-C₅ light components, thereby a higher xylene yield and a high benzene product purity can be achieved.

CN108,948,366A discloses the preparation of a Fe-MOF catalyst with abundant Lewis acid sites and its application in desulfurization. The catalyst is prepared from iron salt and trimesic acid as raw materials to form a Fe-MOF catalyst with abundant Lewis acid sites.

CN112,473,743A discloses a Lewis acid-base bifunctional catalyst, its preparation method and application. The catalyst is a three-dimensional porous structure catalyst formed by assembling mixed ligands and central particles, wherein the mixed ligands are composed of 2,4,6-tris(4-pyridyl)-1,3,5-triazine and trimesic acid, and Ni (II) is used as the central particle. The catalyst has Lewis acid-base pair bifunctional active sites, and can be used for one-pot cascade aromatic alcohol catalytic oxidation Knoevenagel condensation reaction under mild conditions.

CN1,362,286A discloses an alkylation catalyst with an inorganic or organic porous carrier and a heteropoly acid and a Lewis acid as main components. In the catalyst, a heteropoly acid and a Lewis acid are simultaneously supported on a porous carrier, and the heteropoly acid accounts for 10wt%-55wt% of the carrier while the Lewis acid accounts for 1wt%-5wt% of the carrier.

In summary, the current focus on the effect of Lewis acid on catalyst performance is mainly focused on the increase in the amount of acid and its interaction with other active sites, and the hydrocracking catalysts for non-aromatic hydrocarbons known in the prior art still have the problem of insufficient conversion rate of non-aromatic hydrocarbons.

### Contents of the invention

An object of the present application is to provide a solid acid catalyst, and its preparation and use, wherein when the catalyst is used in the hydrocracking reaction of non-aromatic hydrocarbons, it has the advantages of high conversion rate and low side reactions.

In order to achieve above object, in one aspect, the present application provides a solid acid catalyst, comprising:
a) a metal oxide carrier;
b) at least one modifying metal component, wherein the metal in the modifying metal component is selected from the group consisting of metals of Group VB, Group VIB, Group VIII, Group IVA or Group VA, or a combination thereof;
wherein when the catalyst is characterized by solid state nuclear magnetic resonance with trimethylphosphine as the probe molecule, the catalyst exhibits a spectral peak in the range of -5 to -20 ppm.

In another aspect, a method for preparing the catalyst of the present application is provided, comprising:
1) etching a metal oxide carrier raw material with an acidic pH buffer having a pH value of 1-5, to obtain a carrier precursor;
2) washing the carrier precursor obtained in step 1) until the pH of the washing liquid is 6-7, and then drying to obtain the carrier; and
3) loading the modifying metal component onto the carrier obtained in step 2), optionally drying, and calcining under an inert or reducing atmosphere to obtain the catalyst.

In another aspect, the use of the catalyst of the present application in hydrocracking reaction of non-aromatic hydrocarbons is provided.

In yet another aspect, a method for hydrocracking of non-aromatic hydrocarbons is provided, which comprises the step of contacting a feedstock containing a non-aromatic hydrocarbon with the catalyst of the present application in the presence of hydrogen to carry out hydrocracking reaction.

The catalyst of the present application has super strong Lewis acid sites on the surface. When the catalyst is used in the hydrocracking reaction of non-aromatic hydrocarbons, the presence of the acid sites facilitates the stable operation of hydrocracking reaction of non-aromatic hydrocarbons under the synergistic effect of the strong Lewis acid sites and metal sites on the catalyst surface, thereby improving the reaction conversion rate and reducing side reactions. At the same time, the catalyst preparation method of the present application is simple to operate, economically feasible, and easy to industrialize.

Other features and advantages of the present application will be described in detail in the subsequent section of specific embodiments.

### BRIEF DESCRIPTION OF THE DRAWINGS

The accompanying drawings are used to provide a further understanding of the present application and constitute a part of the specification. Together with the following specific embodiments, the drawings are used to explain the present application but do not constitute a limitation to the present application. In the accompanying drawings:
Fig. 1 is ³¹P MAS NMR spectrums of the catalysts obtained in Example 1 and Comparative Examples 1-2; and
Fig. 2 is EPR spectrums of the catalysts obtained in Example 1 and Comparative Example 2.

### SPECIFIC EMBODIMENTS

The specific embodiments of the present application are described in detail below in conjunction with the accompanying drawings. It should be understood that the specific embodiments described here are only used to illustrate and explain the present application, and are not used to limit the present application.

Any specific numerical value disclosed herein (including the endpoints of numerical ranges) is not limited to the exact value of the numerical value, but should be understood to also cover values close to the exact value, such as all possible values within the range of ±5% of the exact value. In addition, for the disclosed numerical ranges, arbitrarily combinations can be made between the endpoint values of the ranges, between the endpoint values and the specific point values in the range, and between the individual specific point values, to obtain one or more new numerical ranges, and these new numerical ranges should also be regarded as specifically disclosed herein.

Unless otherwise specified, the terms used herein have the same meaning as commonly understood by those skilled in the art. If a term has a definition herein and its definition is different from the commonly understood meaning in the art, the definition herein shall prevail.

As known in the art, when a solid acid is characterized by solid state nuclear magnetic resonance with trimethylphosphine as the probe molecule, a catalyst with Bronsted acid sites on the surface will exhibit a spectral peak in the range of 0 ppm to -5 ppm (i.e., the peak value of the spectral peak is in the range of 0 ppm to -5 ppm); while a catalyst with Lewis acid sites will exhibit a spectral peak in the range of -5 ppm to -60 ppm, wherein the closer the spectral peak position is to the low-field direction of -5 ppm, the stronger the acidity of the corresponding Lewis acid sites is (see references: Progress in development and application of solid-state NMR for solid acid catalysis, ZHENG Anmin et. al., Chinese Journal of Catalysis, 2013, vol. 34, No.3, 436-491; Surface acidity of tin dioxide nanomaterials revealed with 31P solid-state NMR spectroscopy and DFT calculation, RSC Advance, 2021, 11, 25004-25009; 31P NMR chemical shifts of phosphorus probes as reliable and practical acidity scales for solid and liquid catalysts, Chemical Reviews, 2017, 117, 12475-12531).

In the present application, strong Lewis acid amount and total Lewis acid amount of the catalyst are measured by pyridine adsorption infrared method, wherein the percentage of the peak area of the absorption peak located near 1450 cm⁻¹ in the infrared spectrum after pyridine desorption at 400°C relative to the peak area of the absorption peak located near 1450 cm⁻¹ in the infrared spectrum after desorption at 200 °C is recorded as the proportion of the strong Lewis acid amount to the total Lewis acid amount.

In the present application, the term "non-aromatic hydrocarbon" has the meaning commonly understood in the art, and generally refers to a hydrocarbon compound that does not contain aromatic ring(s) in its molecular structure, which may be saturated or unsaturated, such as cycloalkanes, acyclic alkanes, and the like.

In the present application, the term "cycloalkane" has the meaning commonly understood in the art, and generally refers to a saturated hydrocarbon compound having one or more carbon rings in its molecular structure, such as cyclohexane, methylcyclopentane, and the like.

In this application, except for the contents explicitly stated, any matters or items not mentioned directly adopt those known in the art and no changes are required. Moreover, any embodiment described herein can be freely combined with one or more other embodiments described herein, and the technical solutions or technical ideas formed thereby are all deemed to be part of the original disclosure or original statement of this application, and should not be regarded as new contents not disclosed or anticipated herein, unless a person skilled in the art considers that the combination is obviously unreasonable.

All patent and non-patent documents, including but not limited to textbooks, journal articles and the like, mentioned herein are incorporated herein by reference in their entirety.

As described above, in a first aspect, the present application provides a solid acid catalyst, comprising:
a) a metal oxide carrier;
b) at least one modifying metal component;
wherein when the catalyst is characterized by solid state nuclear magnetic resonance with trimethylphosphine as the probe molecule, the catalyst exhibits a spectral peak in the range of -5 to -20 ppm, i.e., exhibits at least one spectral peak with peak value in the range of -5 to -20 ppm.

The existing hydrocracking catalysts for non-aromatic hydrocarbons generally use a Brønsted acid and a metal component for synergistic catalysis. However, such catalyst systems often have problems such as low conversion rate, incomplete cracking and the like. The catalyst of the present application exhibits a spectral peak in the range of -5 to -20 ppm in the solid nuclear magnetic resonance spectrum obtained by conducting testing with trimethylphosphine as probe molecule, indicating that the surface of the catalyst has super strong Lewis acid sites. Not limited to a specific theory, the inventors of the present application found that this type of super strong Lewis acid sites has a strong interaction with hydrocarbon reaction molecules in the cracking reaction, so it can activate C-H bonds and C-C bonds, and promote the proceeding of uneven breakage process of these chemical bonds. For the hydrocracking reaction of non-aromatic hydrocarbons, the formation of carbenium ions and the β-breakage process are two important reaction processes, and they are respectively related to the uneven breakage of C-H bonds and C-C bonds. The super strong Lewis acid sites in the catalyst system of the present application can not only promote the occurrence of these processes, but also have a good synergistic effect with the metal components on the catalyst surface. Therefore, the super strong Lewis acid sites of the catalyst of the present application have the effects of accelerating the formation of carbenium ions and promoting the occurrence of the β-breakage process in the hydrocracking reaction of non-aromatic hydrocarbons, which facilitates the stable operation of hydrocracking reaction of the non-aromatic hydrocarbons under the synergistic effect of the strong Lewis acid sites and the metal on the catalyst surface, and in turn increases the conversion rate of the hydrocracking reaction of non-aromatic hydrocarbons and reduces undesirable side reactions.

The catalyst of the present application has uniformly distributed coordination-unsaturated metal ion sites on carrier surface, and the introduced modifying metal can be bound to these coordination-unsaturated sites to form uniformly distributed super strong Lewis acid sites, thereby showing the above-mentioned special solid nuclear magnetic resonance spectrum feature. Without being limited to a specific theory, it is believed that the super strong Lewis acid sites of the catalyst of the present application can be represented by the structure shown in the following formula: 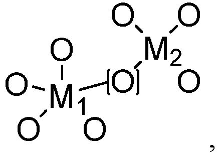 wherein, M₁ is a metal element in a metal oxide carrier, and the number of O atoms coordinating with it depends on the specific metal oxide selected; the number of coordinated oxygen atoms shown in above formula is only schematic; here M₁ is a strong Lewis acid site; M₂ is a modifying metal, and the number of O atoms coordinated with M₂ also depends on the specific metal selected; the number of coordinated oxygen atoms shown in above formula is only schematic; the brackets "[ ]" in above formula indicate that M₁ is the coordination-unsaturated metal ion site.

In certain preferred embodiments, when the catalyst is characterized by solid nuclear magnetic resonance with trimethylphosphine as the probe molecule, the sum of peak areas of the catalyst in the range of -5 to -20 ppm accounts for 1-100% of the sum of peak areas of the catalyst in the range of -5 to -60 ppm, for example 1%, 2%, 5%, 10%, 15%, 20%, 25%, 30%, 35%, 40%, 45%, 50%, 55%, 60%, 65%, 70%, 75%, 80%, 85%, 90%, 95%, 98%, 99%, 100%, or any value within the numerical range constituted by any two of these values, preferably 20-100%, such as 20%, 25%, 30%, 35%, 40%, 45%, 50%, 55%, 60%, 65%, 70%, 75%, 80%, 85%, 90%, 95%, 98%, 99%, or 100%. The above ratios represent the proportion of the super strong Lewis acid amount to the total Lewis acid amount of the catalyst of the present application.

In certain specific embodiments, when the catalyst of the present application is characterized by electron paramagnetic resonance, the catalyst exhibits a spectral peak in the range of g = 1.9 - 2.1, that is, the catalyst exhibits at least one spectral peak with peak value in the range of g = 1.9 - 2.1, indicating there are defect sites formed by coordination-unsaturated sites on the surface of the catalyst.

In a preferred embodiment, when the catalyst is subjected to pyridine adsorption infrared test with pyridine as the probe molecule, the proportion of the strong Lewis acid amount to the total Lewis acid amount in the catalyst is 5-100%, such as 5%, 10%, 15%, 20%, 30%, 40%, 50%, 60%, 70%, 80%, 90% or 100%, preferably 20-100%, and more preferably 40-100%.

According to the present application, it is required that the metal oxide carrier can generate surface coordination-unsaturated metal ion sites after being treated in an acidic environment with a pH value of 1 to 5. In a preferred embodiment, the metal oxide carrier is selected from the group consisting of Al₂O₃, TiO₂, ZrO₂, ZnO, Nb₂O₅, CuO, CeO₂ or a combination thereof, more preferably selected from the group consisting of ZrO₂, Nb₂O₅ or a combination thereof.

According to the present application, the metal in the modifying metal component can be selected from the group consisting of metals of Group VB, Group VIB, Group VIII, Group IVA or Group VA, or a combination thereof. In a preferred embodiment, the metal in the modifying metal component is selected from the group consisting of V, Cr, Mo, W, Co, Sn, Bi or a combination thereof, more preferably Co, W, or a combination thereof.

In a preferred embodiment, based on the weight of the catalyst, the content of the modifying metal component, calculated as metal, is 0.1-50%, such as 0.1%, 0.5%, 1%, 2%, 3%, 4%, 5%, 10%, 15%, 20%, 25%, 30%, 35% or 40%, preferably 0.5-40%, more preferably 1-10%, and the content of the metal oxide carrier, calculated as metal oxide, is 50-99.9%, such as 50%, 55%, 60%, 65%, 70%, 75%, 80%, 85%, 90%, 95%, 96%, 97%, 98% or 99%, preferably 60-99.5%, more preferably 90-99%.

In a specific embodiment, the metal oxide carrier is treated by etching with an acidic pH buffer, wherein the pH value of the acidic pH buffer is preferably 1-5, such as 1.0, 1.5, 2.0, 2.5, 3.0, 3.5, 4.0 or 4.5, more preferably 2-4.

In a specific embodiment, the catalyst is treated with a reducing or inert atmosphere, and the total peak area in g = 1.9 - 2.1 of the catalyst increases by 5-100%, such as, 5%, 10%, 15%, 20%, 25%, 30%, 35%, 40%, 45%, 50%, 55%, 60%, 65%, 70%, 75%, 80%, 85%, 90% or 95%, compared with the catalyst not treated with a reducing or inert atmosphere, when the catalyst is characterized by electron paramagnetic resonance.

In a second aspect, a method for preparing the catalyst of the present application is provided, which comprises:
1) etching a metal oxide carrier raw material with an acidic pH buffer having a pH value of 1-5, preferably 2-4, to obtain a carrier precursor;
2) washing the carrier precursor obtained in step 1) until the pH of the washing liquid is 6-7, and then drying to obtain the carrier; and
3) loading a modifying metal component onto the carrier obtained in step 2), optionally drying, and calcining under an inert or reducing atmosphere to obtain the catalyst.

In a specific embodiment, in the catalyst preparation method of the present application, before the step 3), there is no step of calcining the carrier precursor obtained in step 1) or the carrier obtained in step 2).

In the catalyst preparation method of the present application, super strong Lewis acid sites can be uniformly introduced in the carrier surface by etching the surface of the metal oxide carrier with an acidic buffer solution with a specific pH value range, fully washing and loading the modifying metal component, and then calcining under an inert or reducing atmosphere. Not limited to a specific theory, the inventors of the present application have found through research that, during the metal loading process, the metal precursor tends to bind onto the coordination-unsaturated sites on the carrier surface, and the acidic pH buffer with a pH value of 1-5 can ensure that the carrier is etched in a suitable and stable acidic environment, thereby achieving mild etching of only the atomic layer(s) at the surface and near the surface of the carrier metal oxide(e.g., 1-3 atomic layers at the surface); thereby, uniformly distributed coordination-unsaturated metal ion sites are formed on the carrier surface, and reconstruction of the surface of the carrier metal oxide is not rendered at the same time. After fully washing and loading the modifying metal component, the use of an inert or reducing atmosphere can further stabilize the coordination-unsaturated sites on the carrier surface during the calcination process, thereby facilitating the immobilization of the metal component on the carrier. When the loaded metal is bound to these coordination-unsaturated sites, uniformly distributed super strong Lewis acid sites are formed, and excessive aggregation of Lewis acid sites does not occur, such that the catalyst with super strong Lewis acid sites of the present application is obtained.

There are no strict restrictions on the specific chemical composition of the acidic pH buffer used in present application, as long as it can maintain the desired pH value (i.e., pH value 1-5, such as 1.0, 1.5, 2.0, 2.5, 3.0, 3.5, 4.0 or 4.5, preferably 2-4) during the etching treatment procedure of step 1). Specific examples of the buffer include, but are not limited to, citric acid-sodium citrate buffer, acetic acid-sodium acetate buffer, glycine-hydrochloric acid buffer, citric acid-sodium hydroxide-hydrochloric acid buffer, etc. In a preferred embodiment, the acidic pH buffer used in step 1) is acetic acid-sodium acetate buffer. The using of acidic buffer within the above pH range can not only realize the etching process of the atomic layer(s) at the surface and near the surface of the carrier and introduce coordination-unsaturated metal ion sites, but also avoid the reconstruction of the atomic layer(s) at the surface of the carrier during severe etching process, so that the coordination-unsaturated metal ion sites generated by etching can be uniformly distributed on the surface of the carrier, so as to facilitate the subsequent introduction of modifying metal and the generation of super strong Lewis acid sites.

In a specific embodiment, during the etching treatment process of step 1), the pH value of the acidic pH buffer is maintained at 1-5, such as 1.0, 1.5, 2.0, 2.5, 3.0, 3.5, 4.0 or 4.5, preferably in the range of 2-4. When the pH value of the acidic pH buffer exceeds above range, the super strong Lewis acid sites required by the present application cannot be formed. Specifically, when pH > 5, it is difficult to achieve the etching process on the surface of the carrier; and when pH <1, the etching process is too violent, which will cause reconstruction of the surface of the carrier, even the loss of most of the structure, and thus the coordination-unsaturated metal ion sites cannot be uniformly introduced onto the carrier surface, and super strong Lewis acid sites cannot be formed after loading the modifying metal.

According to the present application, the carrier raw material used in step 1) can be selected from various metal oxides that can generate surface coordination-unsaturated metal ion sites after etching treatment in an acidic environment with a pH value of 1 to 5. In a preferred embodiment, the carrier raw material is selected from the group consisting of Al₂O₃, TiO₂, ZrO₂, ZnO, Nb₂O₅, CuO, CeO₂, or a combination thereof, more preferably selected from the group consisting of ZrO₂, Nb₂O₅, or a combination thereof.

In a preferred embodiment, the mass ratio of the carrier raw material to the acidic pH buffer in step 1) is 1:5 to 1:50, such as 1:5, 1:6, 1:7, 1:8, 1:9, 1:10, 1:15, 1:20, 1:25, 1:30, 1:35, 1:40, 1:45 or 1:50, more preferably 1:10 to 1:20.

In a preferred embodiment, in step 1), the conditions of the etching treatment include: etching temperature of 10-50 °C, such as 10 °C, 15 °C, 20 °C, 25 °C, 30 °C, 35 °C, 40 °C, 45 °C or 50 °C, preferably 15-30 °C, and etching time of 1-20h, such as 1h, 2h, 3h, 4h, 5h, 6h, 7h, 8h, 9h, 10h, 12h, 15h, 18h or 20h, preferably 3-10h. During the etching treatment, the carrier raw material is preferably mixed with the acidic pH buffer to perform the etching treatment. The etching treatment in step 1) of the present application is carried out under relatively mild conditions, which can uniformly increase the coordination-unsaturated sites on the carrier surface; in contrast, etching under violent reaction conditions (such as boiling or near-boiling conditions) or etching for a too long time tends to lead to the destruction of the surface structure of the metal oxide and the loss of the coordination-unsaturated metal ion sites, which in turn is not favorable to the formation of uniformly distributed coordination-unsaturated metal ion sites on carrier surface.

In a preferred embodiment, in step 1), the reaction mixture is filtered after completion of etching to obtain the carrier precursor.

According to the present application, in step 2), the carrier precursor obtained in step 1) is washed until the pH value of the washing liquid is 6-7. Generally, the washing can be carried out with water, but other conventional washing agents, such as ethanol or a mixture of ethanol and water, can also be used. In step 2) of the present application, the residue of the acidic pH buffer can be fully removed by washing the carrier precursor to nearly neutral, thereby avoiding the poisoning of the acid sites on the catalyst surface by the residue. In contrast, directly drying the carrier precursor and conducting subsequent treatments without washing will cause the covering of the coordination-unsaturated metal ion sites on the carrier surface, so that it is difficult for the modifying metal to settle on the coordination-unsaturated metal ion sites, causing the catalyst acid density to drop sharply, and that it is impossible to produce super strong Lewis acid sites.

In a preferred embodiment, the conditions of drying in step 2) include: drying temperature of 50-130 °C, such as 50 °C, 60 °C, 70 °C, 80 °C, 90 °C, 100 °C, 110 °C, 120 °C or 130 °C, preferably 80-120 °C, and drying time of 5-30h, such as 5h, 10h, 12h, 15h, 18h, 20h, 22h, 25h, 28h or 30h, preferably 12-20h.

In a specific embodiment, when the carrier obtained in step 2) is characterized by solid state nuclear magnetic resonance with trimethylphosphine as the probe molecule, it exhibits a spectral peak in the range of -5 to -60 ppm, indicating that coordination-unsaturated sites with Lewis acidity are formed on the surface of the obtained carrier precursor.

According to the present application, before step 3), there is no step of calcining the carrier precursor obtained in step 1) or the carrier obtained in step 2). If the carrier precursor or the carrier is calcined before step 3), the disappearance of coordination-unsaturated metal ion sites generated during the etching process will be caused due to surface reconstruction under high-temperature calcination, and when the modifying metal is loaded, the modifying metal cannot selectively settle on the coordination-unsaturated metal ion sites, so that the catalyst is unable to generate super strong Lewis acid sites.

According to the present application, in step 3), the modifying metal component can be loaded onto the carrier obtained in step 2) by various conventional manners, for example, the loading manner can be impregnation, precipitation or physical kneading, preferably incipient wetness impregnation.

According to the present application, the metal in the modifying metal component used in step 3) can be selected from the group consisting of metals of Group VB, Group VIB, Group VIII, Group IVA or Group VA, or a combination thereof. In a preferred embodiment, the metal in the modifying metal component is selected from the group consisting of V, Cr, Mo, W, Co, Sn, Bi, or a combination thereof, more preferably Co, W, or a combination thereof.

In a preferred embodiment, the modifying metal component is loaded onto the carrier in the form of a metal precursor, and the metal precursor is preferably a salt of the corresponding metal. For example, the precursor of V can be a vanadate, the precursor of Cr can be chromium nitrate or chromium chloride, the precursor of Mo can be a molybdate, the precursor of W can be a tungstate, the precursor of Co can be cobalt nitrate or cobalt chloride, the precursor of Ni can be nickel nitrate or nickel chloride, the precursor of Sn can be tin chloride, and the precursor of Bi can be bismuth nitrate, preferably cobalt nitrate and ammonium tungstate.

In a preferred embodiment, in step 3), the conditions of drying include: drying temperature of 50-130 °C, such as 50 °C, 60 °C, 70 °C, 80 °C, 90 °C, 100 °C, 110 °C, 120 °C or 130 °C, preferably 80-120 °C, and drying time of 5-30h, such as 5h, 10h, 12h, 15h, 18h, 20h, 22h, 25h, 28h or 30h, preferably 12-20h.

According to the present application, in step 3), the carrier after loading the modifying metal component is calcined under a reducing or inert atmosphere, which can stabilize the coordination-unsaturated sites on the carrier surface during the calcination process, thereby facilitating the immobilization of the metal component on the carrier. In contrast, calcination under a non-reducing or non-inert atmosphere (such as an air atmosphere) will lead to reconstruction of the carrier surface, resulting in the loss of coordination-unsaturated metal ion sites, and resulting in the inability to form strong Lewis acid sites. The total peak area in g = 1.9 - 2.1 range of the catalyst of the present application obtained by calcination under a reducing or inert atmosphere increases by at least 5%, compared with the catalyst obtained by calcination under a non-reducing or non-inert atmosphere, when the catalyst is characterized by electron paramagnetic resonance, indicating that the surface of the catalyst of the present application has more defect sites formed by coordination-unsaturated sites.

In a preferred embodiment, in step 3), the conditions of calcination include: calcination temperature of 250-600 °C, for example, 250 °C, 300 °C, 350 °C, 400 °C, 450 °C, 500 °C, 550 °C or 600 °C, preferably 300-500 °C, and calcination time of 1-5h, for example, 1.0 h, 1.5 h, 2.0 h, 2.5 h, 3.0 h, 3.5 h, 4.0 h, 4.5 h or 5.0 h, preferably 2-4 h.

In a preferred embodiment, in step 3), the calcination atmosphere is selected from the group consisting of H₂, N₂, Ar, He, H₂/Ar mixed gas, H₂/N₂ mixed gas, H₂/He mixed gas, or a combination thereof.

According to the present application, the morphology of the carrier in the catalyst does not change significantly before and after etching, calcination and loading. In some embodiments, the specific surface area of the catalyst is slightly higher than that of the original carrier, for example, 0.5-5% higher.

In a third aspect, the use of the catalyst of the present application in hydrocracking reaction of a non-aromatic hydrocarbon is provided.

In a fourth aspect, a method for hydrocracking of a non-aromatic hydrocarbon is provided, which comprises the step of contacting a feedstock containing a non-aromatic hydrocarbon with the catalyst of the present application in the presence of hydrogen to carry out hydrocracking reaction.

In a preferred embodiment, the conditions of the hydrocracking reaction include: reaction temperature of 200-500 °C, reaction pressure of 0.5-6 MPa, hydrogen/hydrocarbon molar ratio of 2-10, and feedstock weight hourly space velocity of 1.0-20.0h⁻¹.

The catalyst of the present application is applicable to hydrocracking reaction of various non-aromatic hydrocarbons, on which the present application has no strict restrictions. In a preferred embodiment, the non-aromatic hydrocarbon is selected from the group consisting of 6 to 9 carbon atoms-containing cycloalkanes, acyclic alkanes, or a combination thereof. More preferably, the non-aromatic hydrocarbon is selected from the group consisting of cyclohexane, methylcyclopentane, or a combination thereof.

In a specific embodiment, the target product of the hydrocracking reaction of non-aromatic hydrocarbon is C₂-C₅ light non-aromatic hydrocarbons.

### Examples

The present application is further described in detail below through examples, but the present application is not limited thereto.

### Testing Methods

In the following Examples and Comparative Examples, the testing method of solid state nuclear magnetic resonance spectrum (³¹P MAS NMR) using trimethylphosphine as the probe molecule is as follows: after activating the sample under vacuum, a certain amount of trimethylphosphine molecules is adsorbed onto the sample to be tested in situ, the sample is loaded in a glove box protected by N₂, and the test is performed using Bruker AVANCE III 400WB solid state nuclear magnetic resonance instrument, with a rotor diameter of 4 mm and a sample rotation speed of 12 kHz. The ³¹P MAS NMR spectrum is tested by high-power proton decoupling, and the test results are calibrated and analyzed using NH₄H₂PO₄ as standard reference.

In the following Examples and Comparative Examples, the electron paramagnetic resonance (EPR) test is carried out using EMX-8/2.7 instrument from Bruker, Germany. The sample is tested at room temperature, at a frequency of 9.88 GHz and at a microwave power of 2.0 mW, and the spectrum is obtained after normalization according to the magnetic field conditions, scanning conditions and the like during testing.

In the following Examples and Comparative Examples, the method for pyridine adsorption infrared testing using pyridine as the probe molecule is as follows: the acidity and acid amount of the sample are determined using Nexsus TM infrared spectrometer (Py-IR) from Nicolet, USA. During the testing, the sample is compressed into a tablet, evacuated to 10⁻⁴ Pa and heated to 400 °C for heating treatment for 2 h; after the temperature dropped to 200 °C, pyridine is statically adsorbed for 1 min, and after balancing for 5 min, low vacuum is applied for 10 min and high vacuum iss applied for 30 min, and IR scanning is performed; after the temperature is raised to 400 °C within 10 min and then balanced for 5 min, IR scanning is performed. The Brønsted acid amount and Lewis acid amount are calculated according to the absorption peak areas of the sample near 1540 cm⁻¹ and 1450 cm⁻¹, respectively. The percentage of the peak area of the absorption peak located near 1450 cm⁻¹ in the infrared spectrum after pyridine desorption at 400°C relative to the peak area of the absorption peak located near 1450 cm⁻¹ in the infrared spectrum after desorption at 200°C is recorded as the proportion of the strong Lewis acid amount to the total Lewis acid amount.

In the following Examples and Comparative Examples, the specific surface areas of the samples are measured by Tristar II 3020 fully-automatic specific surface area and pore analyzer from Micromeritics, USA. After pretreatment, the samples are placed in liquid nitrogen, and the nitrogen adsorption and desorption isotherms are measured at -196°C, and the specific surface areas of the samples are calculated using the BET equation.

### [Example 1]

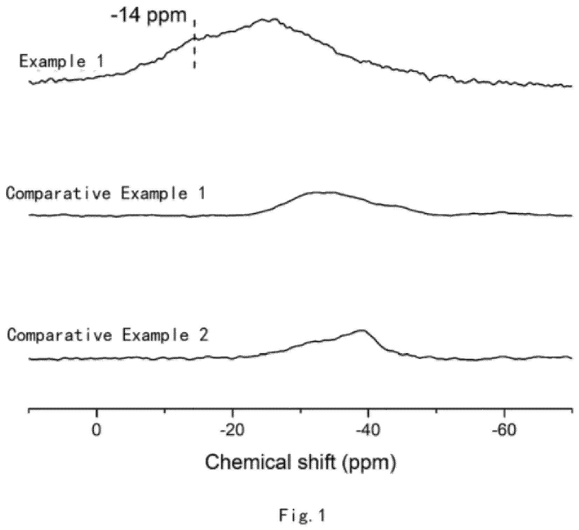

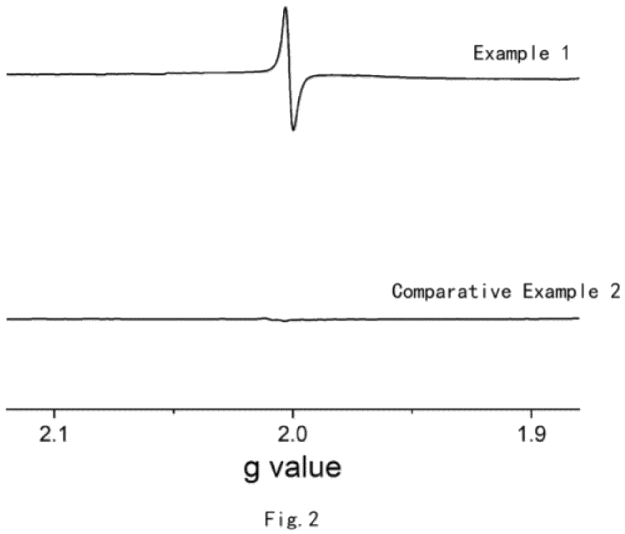

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100°C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A1 with a Co content of 5wt%. The ³¹P MAS NMR characterization result of catalyst A1 was shown in Fig. 1, wherein a spectral peak was shown at -14 (± 1) ppm, which was the signal generated by trimethylphosphine molecules adsorbed by the super strong Lewis acid sites on the catalyst surface. The sum of the peak areas of the catalyst in the range of -5 to -20 ppm accounted for 40% of the sum of the peak areas of the catalyst in the range of -5 to -60 ppm. Catalyst A1 was subjected to adsorption infrared spectrum test using pyridine as the probe molecule. The proportion of the strong Lewis acid amount to the total Lewis acid amount in catalyst A1 was 82%. The EPR characterization result of catalyst A1 was shown in Fig. 2. The sample exhibited a spectral peak at g = 2.002, indicating that the catalyst had defect sites formed by coordination-unsaturated sites. The total peak area of catalyst A1 in the range of g = 1.9-2.1 was increased by about 92% compared with the catalyst calcined in a non-reducing or non-inert atmosphere (Comparative Example 2).

### Catalyst reaction performance test:

10 g of catalyst A1 was placed in a reactor, in which hydrogen was introduced to reduce at 380 °C for 3 hours; the temperature was lowered to 350 °C, and then hydrogen and a mixture of 50 wt% cyclohexane and 50 wt% methylcyclopentane were introduced to react. The reaction conditions were as follows: total weight hourly space velocity of 5 h⁻¹, reaction temperature of 350 °C, reaction pressure of 5 MPa, and hydrogen/hydrocarbon molar ratio of 3. The test results were shown in Table 1.

### [Example 2]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 3 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A2 with a Co content of 5wt%. The ³¹P MAS NMR spectrum of catalyst A2 exhibited a spectral peak at -14 (±1) ppm. Catalyst A2 was subjected to adsorption infrared spectrum test using pyridine as the probe molecule. The proportion of the strong Lewis acid amount to the total Lewis acid amount in catalyst A2 was 56%. The EPR characterization result of Catalyst A2 was similar to that of Example 1, and its total peak area in the range of g = 1.9-2.1 was increased by about 83% compared with the catalyst of Comparative Example 2.

The reaction performance test of the catalyst was carried out with reference to Example 1. The test results were shown in Table 1.

### [Example 3]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A3 with a Co content of 2wt%. The ³¹P MAS NMR spectrum of catalyst A3 exhibited a spectral peak at -14 (±1) ppm. Catalyst A3 was subjected to adsorption infrared spectrum test using pyridine as the probe molecule. The proportion of the strong Lewis acid amount to the total Lewis acid amount in catalyst A3 was 42%. The EPR characterization result of Catalyst A3 was similar to that of Example 1, and its total peak area in the range of g = 1.9-2.1 was increased by about 95% compared with the catalyst of Comparative Example 2.

The reaction performance test of the catalyst was carried out with reference to Example 1. The test results were shown in Table 1.

### [Example 4]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of ammonium tungstate, and dried at 100 °C for 15 hours. The obtained product was calcined at 400 °C in an Ar atmosphere for 3 hours to obtain catalyst A4 with a W content of 5wt%. The ³¹P MAS NMR spectrum of catalyst A4 exhibited a spectral peak at -14 (±1) ppm. Catalyst A4 was subjected to adsorption infrared spectrum test using pyridine as the probe molecule. The proportion of the strong Lewis acid amount to the total Lewis acid amount in catalyst A4 was 79%. The EPR characterization result of catalyst A4 was similar to that of Example 1, and its total peak area in the range of g = 1.9-2.1 was increased by about 90% compared with the catalyst of Comparative Example 2.

The reaction performance test of the catalyst was carried out with reference to Example 1. The test results were shown in Table 1.

### [Example 5]

10 g of Nb₂O₅ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A5 with a Co content of 5wt%. The ³¹P MAS NMR spectrum of catalyst A5 exhibited a spectral peak at -14 (±1) ppm. Catalyst A5 was subjected to adsorption infrared spectrum test using pyridine as the probe molecule. The proportion of the strong Lewis acid amount to the total Lewis acid amount in catalyst A5 was 75%. The EPR characterization result of catalyst A5 was similar to that of Example 1, and its total peak area in the range of g = 1.9-2.1 was increased by about 89% compared with the catalyst calcined in a non-reducing or non-inert atmosphere.

The reaction performance test of the catalyst was carried out with reference to Example 1. The test results were shown in Table 1.

### [Example 6]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 1 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A6 with a Co content of 5wt%. The ³¹P MAS NMR spectrum of catalyst A6 exhibited a spectral peak at -14 (±1) ppm. Catalyst A6 was subjected to adsorption infrared spectrum test using pyridine as the probe molecule. The proportion of the strong Lewis acid amount to the total Lewis acid amount in catalyst A6 was 17%. The EPR characterization result of catalyst A6 was similar to that of Example 1, and its total peak area in the range of g = 1.9-2.1 was increased by about 21% compared with the catalyst of Comparative Example 2.

The reaction performance test of the catalyst was carried out with reference to Example 1. The test results were shown in Table 1.

### [Example 7]

10 g of ZrO₂ was taken and etched in a citric acid-sodium citrate buffer solution with a pH of 4.8. The mass ratio of the carrier to the citric acid-sodium citrate buffer solution was 1:15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A7 with a Co content of 5wt%. The ³¹P MAS NMR spectrum of catalyst A7 exhibited a spectral peak at -14 (± 1) ppm, which was the signal generated by trimethylphosphine molecules adsorbed by the strong Lewis acid sites on the catalyst surface. Catalyst A7 was subjected to adsorption infrared spectrum test using pyridine as the probe molecule. The proportion of the strong Lewis acid amount to the total Lewis acid amount in catalyst A7 was 12%. The EPR characterization result of catalyst A7 was similar to that of Example 1, and its total peak area in the range of g = 1.9-2.1 was increased by about 15% compared with the catalyst of Comparative Example 2 which was not treated by reducing or inert atmosphere.

The reaction performance test of the catalyst was carried out with reference to Example 1. The test results were shown in Table 1.

### [Example 8]

10 g of ZnO was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A8 with a Co content of 5wt%. The ³¹P MAS NMR spectrum of catalyst A8 exhibited a spectral peak at -14 (±1) ppm. Catalyst A8 was subjected to adsorption infrared spectrum test using pyridine as the probe molecule. The proportion of the strong Lewis acid amount to the total Lewis acid amount in catalyst A8 was 15%. The EPR characterization result of catalyst A8 was similar to that of Example 1, and its total peak area in the range of g = 1.9-2.1 was increased by about 19% compared with the catalyst calcined in a non-reducing or non-inert atmosphere.

The reaction performance test of the catalyst was carried out with reference to Example 1. The test results were shown in Table 1.

### [Example 9]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of chromium nitrate, and dried at 100 °C for 15 hours. The obtained product was calcined at 400 °C in an Ar atmosphere for 3 hours to obtain catalyst A9 with a Cr content of 5wt%. The ³¹P MAS NMR spectrum of catalyst A9 exhibited a spectral peak at -14 (±1) ppm. Catalyst A9 was subjected to adsorption infrared spectrum test using pyridine as the probe molecule. The proportion of the strong Lewis acid amount to the total Lewis acid amount in catalyst A9 was 9%. The EPR characterization result of catalyst A9 was similar to that of Example 1, and its total peak area in the range of g = 1.9-2.1 was increased by about 80% compared with the catalyst of Comparative Example 2.

The reaction performance test of the catalyst was carried out with reference to Example 1. The test results were shown in Table 1.

### [Example 10]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A10 with a Co content of 0.5wt%. The ³¹P MAS NMR spectrum of catalyst A10 exhibited a spectral peak at -14 (±1) ppm. Catalyst A10 was subjected to adsorption infrared spectrum test using pyridine as the probe molecule. The proportion of the strong Lewis acid amount to the total Lewis acid amount in catalyst A10 was 36%. The EPR characterization result of catalyst A10 was similar to that of Example 1, and its total peak area in the range of g = 1.9-2.1 was increased by about 98% compared with the catalyst of Comparative Example 2.

The reaction performance test of the catalyst was carried out with reference to Example 1. The test results were shown in Table 1.

### [Example 11]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in a H₂ atmosphere for 3 hours to obtain catalyst A11 with a Co content of 5wt%. The ³¹P MAS NMR spectrum of catalyst A11 exhibited a spectral peak at -14 (±1) ppm. Catalyst A11 was subjected to adsorption infrared spectrum test using pyridine as the probe molecule. The proportion of the strong Lewis acid amount to the total Lewis acid amount in catalyst A11 was 80%. The EPR characterization result of catalyst A11 was similar to those of Example 1, and its total peak area in the range of g = 1.9-2.1 was increased by about 96% compared with the catalyst of Comparative Example 2.

The reaction performance test of the catalyst was carried out with reference to Example 1. The test results were shown in Table 1.

### [Example 12]

10 g of ZrO₂ was taken and etched in a citric acid-sodium hydroxide-hydrochloric acid buffer solution with a pH of 4.3. The mass ratio of the carrier to the citric acid-sodium hydroxide-hydrochloric acid buffer solution was 1:15. After etching at 18 °C for 3 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A12 with a Co content of 5wt%. The ³¹P MAS NMR spectrum of catalyst A12 exhibited a spectral peak at -14 (±1) ppm. Catalyst A12 was subjected to adsorption infrared spectrum test using pyridine as the probe molecule. The proportion of the strong Lewis acid amount to the total Lewis acid amount in catalyst A12 was 13%. The EPR characterization result of Catalyst A12 was similar to that of Example 1, and its total peak area in the range of g = 1.9-2.1 was increased by about 17% compared with the catalyst of Comparative Example 2.

The reaction performance test of the catalyst was carried out with reference to Example 1. The test results were shown in Table 1.

### [Example 13]

10 g of ZrO₂ was taken and etched in an acetic acid-sodium acetate buffer solution with a pH of 3.8. The mass ratio of the carrier to the acetic acid-sodium acetate buffer solution was 1:15. After etching at 28 °C for 5 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst A13 with a Co content of 5wt%. The ³¹P MAS NMR spectrum of catalyst A13 exhibited a spectral peak at -14 (±1) ppm. Catalyst A13 was subjected to adsorption infrared spectrum test using pyridine as the probe molecule. The proportion of the strong Lewis acid amount to the total Lewis acid amount in catalyst A13 was 78%. The EPR characterization result of catalyst A13 was similar to that of Example 1, and its total peak area in the range of g = 1.9-2.1 was increased by about 86% compared with the catalyst of Comparative Example 2.

The reaction performance test of the catalyst was carried out with reference to Example 1. The test results were shown in Table 1.

### [Comparative Example 1]

10 g of ZrO₂ was taken and incipient wetness impregnated with a certain amount of cobalt nitrate, and dried at 100 °C for 15 hours. The obtained product was calcined at 400 °C in Ar atmosphere for 3 hours to obtain catalyst DA1 with a Co content of 5wt%. No spectral peak was observed in the range of -5 to -20 ppm in the ³¹P MAS NMR spectrum of catalyst DA1. The sum of the peak areas of the catalyst in the range of -5 to -20 ppm accounted for 0% of the sum of the peak areas of the catalyst in the range of -5 to -60 ppm. Catalyst DA1 was subjected to adsorption infrared spectrum test using pyridine as the probe molecule. The proportion of the strong Lewis acid amount to the total Lewis acid amount in catalyst DA1 was 2%.

The reaction performance test of the catalyst was carried out with reference to Example 1. The test results were shown in Table 1.

### [Comparative Example 2]

10 g of ZrO₂ was taken and etched in an acetic acid-sodium acetate buffer solution with a pH of 3.8. The mass ratio of the carrier raw material to the acetic acid-sodium acetate buffer solution was 1: 15. After etching at 18 °C for 8 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an air atmosphere for 3 hours to obtain catalyst DA2 with a Co content of 5wt%. No spectral peak was observed in the range of -5 to -20 ppm in the ³¹P MAS NMR spectrum of catalyst DA2. The sum of the peak areas of the catalyst in the range of -5 to -20 ppm accounted for 0% of the sum of the peak areas of the catalyst in the range of -5 to -60 ppm. Catalyst DA2 was subjected to adsorption infrared spectrum test using pyridine as the probe molecule. The proportion of the strong Lewis acid amount to the total Lewis acid amount in catalyst DA2 was 3%. The EPR characterization result of Catalyst DA2 was shown in Fig. 2. Obvious signal peak(s) of oxygen defects was not observed in the range of g = 1.9-2.1.

The reaction performance test of the catalyst was carried out with reference to Example 1. The test results were shown in Table 1.

### [Comparative Example 3]

10 g of ZrO₂ was taken and etched in an acetic acid-sodium acetate buffer solution with a pH of 3.8. The mass ratio of the carrier to the acetic acid-sodium acetate buffer solution was 1: 15. After etching at 18 °C for 8 hours, a carrier precursor was obtained after suction filtration. The carrier precursor was dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst DA3 with a Co content of 5wt%. No spectral peak was observed in the range of -5 to -20 ppm in the ³¹P MAS NMR spectrum of catalyst DA3. Catalyst DA3 was subjected to adsorption infrared spectrum test using pyridine as the probe molecule. The proportion of the strong Lewis acid amount to the total Lewis acid amount in catalyst DA3 was 1%.

The reaction performance test of the catalyst was carried out with reference to Example 1. The test results were shown in Table 1.

### [Comparative Example 4]

10 g of ZrO₂ was taken and etched in an acetic acid-sodium acetate buffer solution with a pH of 3.8. The mass ratio of the carrier to the acetic acid-sodium acetate buffer solution was 1: 15. After treating at 90 °C for 30 hours, a carrier precursor was obtained after suction filtration. The carrier precursor was washed with deionized water until the pH of the washing liquid was 7, and dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst DA4 with a Co content of 5wt%. No spectral peak was observed in the range of -5 to -20 ppm in the ³¹P MAS NMR spectrum of catalyst DA4. Catalyst DA4 was subjected to adsorption infrared spectrum test using pyridine as the probe molecule. The proportion of the strong Lewis acid amount to the total Lewis acid amount in catalyst DA4 was 2%.

The reaction performance test of the catalyst was carried out with reference to Example 1. The test results were shown in Table 1.

### [Comparative Example 5]

10g of WO₃ was taken and calcined at 400 °C in an Ar atmosphere for 3 hours to obtain catalyst DA5.

No spectral peak was observed in the range of -5 to -20 ppm in the ³¹P MAS NMR spectrum of catalyst DA5. Catalyst DA5 was subjected to adsorption infrared spectrum test using pyridine as the probe molecule. The proportion of the strong Lewis acid amount to the total Lewis acid amount in catalyst DA5 was 40%.

The reaction performance test of the catalyst was carried out with reference to Example 1. The test results were shown in Table 1.

### [Comparative Example 6]

10 g of ZrO₂ was taken and etched in a hydrochloric acid solution with a pH of 0.1. The mass ratio of the carrier to the hydrochloric acid solution was 1:15. After etching at 18 °C for 8 hours, a carrier precursor was obtained after suction filtration. The carrier precursor was washed with deionized water until the pH of the washing liquid was 7, and the carrier precursor was dried at 100 °C for 15 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst DA6 with a Co content of 5wt%. No spectral peak was observed in the range of -5 to -20 ppm in the ³¹P MAS NMR spectrum of catalyst DA6. Catalyst DA6 was subjected to adsorption infrared spectrum test using pyridine as the probe molecule. The proportion of the strong Lewis acid amount to the total Lewis acid amount in catalyst DA6 was 0.5%.

The reaction performance test of the catalyst was carried out with reference to Example 1. The test results were shown in Table 1.

### [Comparative Example 7]

10 g of ZrO₂ was taken and etched in an acetic acid-sodium acetate buffer solution with a pH of 3.8. The mass ratio of the carrier to the acetic acid-sodium acetate buffer solution was 1:15. After etching at 18 °C for 8 hours, a carrier precursor was obtained after suction filtration. The carrier precursor was washed with deionized water until pH of the washing liquid was 7, and dried at 100 °C for 15 hours and calcined at 400 °C for 3 hours to obtain the carrier. The obtained carrier was incipient wetness impregnated with a certain amount of cobalt nitrate, and dried at 100 °C for 15 hours. The obtained product was calcined at 400 °C in an Ar atmosphere for 3 hours to obtain catalyst DA7 with a Co content of 5wt%. No spectral peak was observed in the range of -5 to -20 ppm in the ³¹P MAS NMR spectrum of catalyst DA7. Catalyst DA7 was subjected to adsorption infrared spectrum test using pyridine as the probe molecule. The proportion of the strong Lewis acis amount to the total Lewis acid amount in catalyst DA7 was 2%.

The reaction performance test of the catalyst was carried out with reference to Example 1. The test results were shown in Table 1.

**Table 1. Test results of Examples 1-13 and Comparative Examples 1-7**

| catalyst | carrier | buffer solution (pH, time) | catalyst calcination atmosphere | metal (content) | strong Lewis acid amount/total Lewis acid amount | BET specific surface area increment (%) | cyclohexane conversion rate^{b} | methylcyclopetane conversion rate^{c} | C₂-C₅ yield |
|---|---|---|---|---|---|---|---|---|---|
| Example 1 | ZrO₂ | acetic acid-sodium acetate (3.8, 8h) | Ar | Co (5wt%) | 82% | 4.6 | 99% | 91% | 90% |
| Example 2 | ZrO₂ | acetic acid-sodium acetate (3.8, 3h) | Ar | Co (5wt%) | 56% | 4.8 | 86% | 81% | 77% |
| Example 3 | ZrO₂ | acetic acid-sodium acetate (3.8, 8h) | Ar | Co (2wt%) | 42% | 3.7 | 82% | 75% | 71% |
| Example 4 | ZrO₂ | acetic acid-sodium acetate (3.8, 8h) | Ar | W (5wt%) | 79% | 4.5 | 95% | 89% | 86% |
| Example 5 | Nb₂O₅ | acetic acid-sodium acetate (3.8, 8h) | Ar | Co (5wt%) | 75% | 3.1 | 91% | 83% | 81% |
| Example 6 | ZrO₂ | acetic acid-sodium acetate (3.8, 1h) | Ar | Co (5wt%) | 17% | 1.2 | 35% | 32% | 28% |
| Example 7 | ZrO₂ | citric acid-sodium citrate (4.8, 8h) | Ar | Co (5wt%) | 12% | 0.9 | 32% | 28% | 23% |
| Example 8 | ZnO | acetic acid-sodium acetate (3.8, 8h) | Ar | Co (5wt%) | 15% | 1.0 | 34% | 31% | 26% |
| Example 9 | ZrO₂ | acetic acid-sodium acetate (3.8, 8h) | Ar | Cr (5wt%) | 9% | 4.5 | 26% | 25% | 18% |
| Example 10 | ZrO₂ | acetic acid-sodium acetate (3.8, 8h) | Ar | Co (0.5wt%) | 36% | 4.6 | 75% | 70% | 68% |
| Example 11 | ZrO₂ | acetic acid-sodium acetate (3.8, 8h) | H₂ | Co (5wt%) | 80% | 4.5 | 96% | 89% | 88% |
| Example 12 | ZrO₂ | citric acid-sodium hydroxide-hydrochloric acid (4.3, 8h) | Ar | Co (5wt%) | 13% | 1.5 | 34% | 30% | 25% |
| Example 13 | ZrO₂ | acetic acid-sodium acetate (3.8, 5h, 28°C, etching) | Ar | Co (5wt%) | 78% | 48 | 92% | 87% | 83% |
| Comparative Example 1 | ZrO₂ | None | Ar | Co (5wt%) | 2% | 0.1 | 5% | 4% | 2% |
| Comparative Example 2 | ZrO₂ | acetic acid-sodium acetate (3.8, 8h) | Air | Co (5wt%) | 3% | -0.5 | 7% | 5% | 4% |
| Comparative Example 3 | ZrO₂ | acetic acid-sodium acetate (3.8, 8h, without washing) | Ar | Co (5wt%) | 1% | -0.8 | 3% | 2% | 1% |
| Comparative Example 4 | ZrO₂ | acetic acid-sodium acetate (3.8, 30h, 90°C treatment) | Ar | Co (5wt%) | 2% | 15 | 4% | 3% | 1% |
| Comparative Example 5 | WO₃ | None | Ar | None | 40% | 0.1 | 2% | 2% | 1% |
| Comparative Example 6 | ZrO₂ | hydrochloric acid (0.1, 8h) | Ar | Co (5wt%) | 0.5% | 20 | 2% | 1% | 1% |
| Comparative Example 7 | ZrO₂ | acetic acid-sodium acetate (3.8, 8h, calcining treatment before loading) | Ar | Co (5wt%) | 2% | 0.2 | 3% | 3% | 2% |

| | | | | | | | | | |
|---|---|---|---|---|---|---|---|---|---|
| a. BET specific surface area merement (%) = (BET specific surface area of catalyst - BET specific surface area of original camer) / BET specific surface area of original carrier × 100% b. Cyclohexane conversion rate (%) = Cyclohexane concentration consumed by reaction / Cyclohexane concentration in reactant × 100% c. Methylcyclopentane conversion rate (%) = Methylcyclopentane concentration consumed by reaction / Methylcyclopentane concentration in reactant × 100% | | | | | | | | | |

As shown by the test results in Table 1, the catalysts of Examples 1-13 can significantly improve the conversion rate of feedstock cyclohexane and methylcyclopentane and the yield of C2-C5 target products, compared with the catalysts of Comparative Examples 1-7.

The preferred embodiments of the present application are described in detail above; however, the present application is not limited to the specific details in above embodiments. Within the technical concepts of the present application, a variety of simple modifications can be made to the technical solutions of the present application, and these simple modifications all fall within the protection scope of the present application.

Moreover, it should be noted that the various specific technical features described in the above specific embodiments can be combined in any suitable manner in the absence of contradiction. In order to avoid unnecessary repetition, the various possible combinations are not individually described in the present application.

In addition, the various embodiments of the present application may be arbitrarily combined, and as long as they do not depart the concepts of the present application, they should also be regarded as the contents invented by the present application.

## Claims

1. A solid acid catalyst comprising:
a) a metal oxide carrier, wherein the metal oxide carrier is preferably selected from the group consisting of Al₂O₃, TiO₂, ZrO₂, ZnO, Nb₂O₅, CuO, CeO₂ or a combination thereof;
b) at least one modifying metal component, wherein the metal in the modifying metal component is selected from the group consisting of metals of Group VB, Group VIB, Group VIII, Group IVA or Group VA, or a combination thereof, preferably selected from the group consisting of V, Cr, Mo, W, Co, Sn, Bi or a combination thereof;
wherein when the catalyst is **characterized by** solid state nuclear magnetic resonance with trimethylphosphine as the probe molecule, the catalyst exhibits a spectral peak in the range of -5 to -20 ppm.

2. The catalyst according to claim 1, wherein when the catalyst is subjected to pyridine adsorption infrared test with pyridine as the probe molecule, the proportion of strong Lewis acid amount to total Lewis acid amount in the catalyst is 5-100%, preferably 20-100%, and more preferably 40-100%.

3. The catalyst according to any one of the preceding claims, wherein the metal oxide carrier is selected from the group consisting of ZrO₂, Nb₂O₅ or a combination thereof; and/or the metal in the modifying metal component is selected from the group consisting of Co, W or a combination thereof.

4. The catalyst according to any one of the preceding claims, wherein, based on the weight of the catalyst, the content of the modifying metal component, calculated as metal, is 0.1-50%, preferably 0.5-40%, more preferably 1-10%, and the content of the metal oxide carrier, calculated as metal oxide, is 50-99.9%, preferably 60-99.5%, more preferably 90-99%.

5. The catalyst according to any one of the preceding claims, wherein:
when the catalyst is **characterized by** solid state nuclear magnetic resonance with trimethylphosphine as the probe molecule, the sum of peak areas of the catalyst in the range of -5 to -20 ppm accounts for 1-100%, preferably 20-100% of the sum of peak areas of the catalyst in the range of -5 to -60 ppm; and/or
when the catalyst is **characterized by** electron paramagnetic resonance, the catalyst exhibits a spectral peak in the range of g = 1.9-2.1.

6. A method for preparing the catalyst according to any one of the preceding claims, comprising:
1) etching a metal oxide carrier raw material with an acidic pH buffer having a pH value of 1-5, preferably 2-4, to obtain a carrier precursor;
2) washing the carrier precursor obtained in step 1) until the pH of the washing liquid is 6-7, and then drying to obtain the carrier; and
3) loading the modifying metal component onto the carrier obtained in step 2), optionally drying, and calcining under an inert or reducing atmosphere to obtain the catalyst.

7. The method according to claim 6, wherein before the step 3), there is no step of calcining the carrier precursor obtained in step 1) or the carrier obtained in step 2).

8. The method according to claim 6 or 7, wherein the acidic pH buffer used in step 1) is selected from the group consisting of citric acid-sodium citrate buffer, acetic acid-sodium acetate buffer, glycine-hydrochloric acid buffer, citric acid-sodium hydroxide-hydrochloric acid buffer, preferably acetic acid-sodium acetate buffer.

9. The method according to any one of claims 6 to 8, wherein the carrier raw material used in step 1) is selected from the group consisting of Al₂O₃, TiO₂, ZrO₂, ZnO, Nb₂O₅, CuO, CeO₂ or a combination thereof, preferably selected from the group consisting of ZrO₂, Nb₂O₅ or a combination thereof;
preferably, in step 1), the mass ratio of the carrier raw material to the acidic pH buffer is 1:5 to 1:50, preferably 1:10 to 1:20.

10. The method according to any one of claims 6 to 9, wherein in step 1), the conditions of the etching include: etching temperature of 10-50 °C, preferably 15-30 °C, and etching time of 1-20h, preferably 3-10h.

11. The method according to any one of claims 6 to 10, wherein in step 2), the conditions of the drying include: drying temperature of 50-130 °C, preferably 80-120 °C, and drying time of 5-30h, preferably 12-20h.

12. The method according to any one of claims 6 to 11, wherein in step 3), the conditions of the drying include: drying temperature of 50-130 °C, preferably 80-120 °C, and drying time of 5-30h, preferably 12-20h; and/or,
the conditions of the calcining include: calcination temperature of 250-600 °C, preferably 300-500 °C, calcination time of 1-5h, preferably 2-4h; and calcination atmosphere being selected from the group consisting of H₂, N₂, Ar, He, H₂/Ar mixed gas, H₂/N₂ mixed gas, H₂/He mixed gas, or a combination thereof.

13. Use of the catalyst according to any one of claims 1 to 5 in hydrocracking reaction of a non-aromatic hydrocarbon.

14. A method for hydrocracking of a non-aromatic hydrocarbon, comprising the step of contacting a feedstock containing the non-aromatic hydrocarbon with the catalyst according to any one of claims 1 to 5 in the presence of hydrogen to carry out hydrocracking reaction.

15. The use according to claim 13 or the method according to claim 14, wherein the conditions of the hydrocracking reaction include: reaction temperature of 200-500 °C, reaction pressure of 0.5-6 MPa, hydrogen/hydrocarbon molar ratio of 2-10, and feedstock weight hourly space velocity of 1.0-20.0 h⁻¹.

16. The use according to claim 13 or the method according to claim 14, wherein the non-aromatic hydrocarbon is selected from the group consisting of 6 to 9 carbon atoms-containing cycloalkanes, acyclic alkanes, or a combination thereof, preferably, the non-aromatic hydrocarbon is selected from the group consisting of cyclohexane, methylcyclopentane, or a combination thereof.
